# EUROPÄISCHE PATENTANMELDUNG

(11) **EP 0 602 543 A1**
(43) Veröffentlichungstag der Anmeldung: **22.06.1994**
(21) Anmeldenummer: 93119842.8
(22) Anmeldetag: 09.12.1993
(51) Int. Cl.: A61K 9/70

(54) **Beschichteter Träger zur Diagnose und Therapie von Erkrankungen sowie Verwendung des Trägers**

(30) Priorität: 12.12.1992 DE 4242040
(71) Anmelder: Hauser, Willy E., D-71296 Heimsheim (DE)
(72) Erfinder: Velhover, Ewgenij, GUS 129626 Moskau (RU)
(74) Vertreter: Patentanwälte Ruff, Beier, Schöndorf und Mütschele

(57) **Zusammenfassung**

Bei einem beschichteten Träger zur Diagnose und/oder Therapie von Erkrankungen eines Organismus ist ein Trägermaterial mit mindestens einem Element des Periodensystems oder einer Verbindung dieses Elements beschichtet, wobei das Element in elementarer oder in gebundener Form im Organismus vorkommt. Bei dem Trägermaterial kann es sich beispielsweise um ein Plättchen aus transparentem Kunststoffmaterial handeln. Bei den Elementen des Periodensystems handelt es sich vorzugsweise um sogenannte Makroelemente oder um sogenannte Spurenelemente. Dabei kommen insbesondere Metalle oder Elemente mit zumindest teilweise metallischem Charakter in Frage. Die Erfindung betrifft weiterhin medizinische Diagnostika oder Therapeutika, die die beschriebenen beschichteten Träger aufweisen, sowie die Verwendung des beschichteten Trägers zur Diagnose und/oder Therapie von Erkrankungen eines Organismus, insbesondere des menschlichen Organismus.

## Beschreibung

Die Erfindung betrifft einen beschichteten Träger zur Diagnose und/oder Therapie von Erkrankungen eines Organismus.

Nach neuen Methoden zur Diagnose oder Therapie von Erkrankungen eines Organismus wird ständig geforscht. Dabei handelt es sich um Erkrankungen im weitesten Sinne bei beliebigen Organismen, wie beispielsweise bei Tieren, beim Menschen oder auch bei Pflanzen. Die größte Bedeutung besitzt dabei selbstverständlich die Forschung zur Diagnose und Therapie von Erkrankungen im menschlichen Organismus.

Eine Vielzahl von Erkrankungen hängen mit einem Mangel bestimmter Elemente oder Verbindungen dieser Elemente im Organismus zusammen oder sie äußern sich in einem solchen Mangel. Es gab deshalb bereits Versuche, das Auftreten bestimmter Elemente beispielsweise im menschlichen Organismus quantitativ zu erfassen. So ist eine Methode zur Diagnose von Schwermetallvergiftungen bekannt, bei der die Schwermetalle Cobalt und Nickel in den menschlichen Haaren quantitativ bestimmt werden. Mit dieser Methode ist es jedoch nicht möglich, sich ein vollständiges Bild über das Auftreten von Elementen im Organismus zu verschaffen. Dies liegt unter anderem daran, daß sich Haare innerhalb eines Zeitraums von ungefähr 30 bis 40 Tagen erneuern und eine Bestimmung vieler Elemente, wie Metallen, in den Haaren beispielsweise des Menschen schwierig oder unmöglich ist.

Die Erfindung stellt sich deshalb die Aufgabe, neue Diagnose- und Therapiemittel zur Verfügung zu stellen, die die Diagnose und Therapie einer Vielzahl von Erkrankungen eines Organismus erleichtern oder möglich machen. Diese Mittel sollen sich den Umstand zunutze machen, daß viele Erkrankungen mit einem Mangel bestimmter Elemente im Organismus zusammenhängen. Die Mittel sollen dabei möglichst einfach herstellbar und applizierbar sein, so daß sie beispielsweise auch für eine präklinische Diagnostik und eine sich daran anschließende Korrektur von Erkrankungen geeignet sind.

Diese Aufgabe wird durch einen beschichteten Träger (Unterlage) gelöst, bei dem ein Trägermaterial mit mindestens einem Element des Periodensystems oder einer Verbindung dieses Elements beschichtet ist. Dabei kommt das zur Beschichtung verwendete Element in elementarer oder in gebundener Form im Organismus vor.

Bei der Erfindung wird das als Beschichtung aufgebrachte Element bei der Applikation des Trägers, beispielsweise auf die Haut des Menschen, resorbiert. Besteht im Organismus ein Mangel an diesem Element, so wird das Element über die Körperhaut vom Organismus aufgenommen und die Beschichtung auf dem Trägermaterial wird ganz oder teilweise abgebaut. Besteht kein Mangel an dem Element, so verbleibt die Beschichtung unverändert auf dem Trägermaterial. Gegebenenfalls läßt sich ein erkannter Mangel an einem oder mehreren Elementen gezielt durch Applikation von mit diesen Elementen beschichteten Trägern behandeln. Die Träger sind somit geeignet, sowohl die Bilanz der Elemente im Organismus zu bestimmen, als auch gegebenenfalls diese Bilanz, insbesondere durch Verabreichung bestimmter Dosen, zu korrigieren. Selbstverständlich ist es auch möglich, den mit Hilfe der Erfindung erkannten Mangel an den Elementen durch andere Therapiemaßnahmen wie beispielsweise orale Verabreichung oder Inhalation der Elemente oder ihrer Verbindungen zu korrigieren. Die Erfindung läßt sich sehr gut in entsprechende Diagnose- und/oder Therapieabläufe integrieren.

Es ist bevorzugt, wenn es sich bei dem Trägermaterial um einen flachen Gegenstand handelt, dessen Dicke im Vergleich zu seinen übrigen Abmessungen klein ist. So kann es sich beispielsweise bei dem Trägermaterial um Plättchen handeln, auf die die Beschichtung aus dem Element oder seiner Verbindung, insbesondere einseitig aufgebracht ist. Geeignete Plättchen sind beispielsweise rechteckig und besitzen Abmessungen von 10 x 15 mm und eine Dicke von 0,5 mm. Selbstverständlich ist die Erfindung auf diese Maße des Trägermaterials nicht beschränkt. Je nach gewünschter Verwendung können das Trägermaterial und damit die beschichteten Träger beliebige Abmessungen aufweisen. So ist es beispielsweise zweckmäßig, bei Durchführung einer Therapie größere Träger zu verwenden, da auf diese Weise ein Mangel an bestimmten Elementen durch die größere Aufnahmefläche schneller ausgeglichen werden kann. Außerdem ist bei der Therapie, wenn der Mangel bereits erkannt ist, vergleichsweise unwichtig, in welchem Verhältnis das applizierte Element zu anderen Elementen aufgenommen wird, wie dies bei der Diagnose bestimmt werden soll.

Vorzugsweise ist das Trägermaterial mindestens teilweise transparent. Dabei kann das Trägermaterial insbesondere aus transparentem oder "klarem" Kunststoff gebildet sein. Ein solcher geeigneter Kunststoff ist beispielsweise Polyethylen. Ein solches transparentes Trägermaterial ist insbesondere dann von Vorteil, wenn optische Nachweismethoden verwendet werden, um zu bestimmen, wieviel der Beschichtung und damit des betreffenden Elements vom Organismus aufgenommen wurde.

In diesem Zusammenhang sei erwähnt, daß die Aufnahme des Elements vom Organismus und damit das Verschwinden der Beschichtung des Trägers auf jede geeignete Weise am Träger bestimmt werden kann. Eine besonders vorteilhafte Methode ist dabei ein optischer Nachweis. Im einfachsten Fall kann dieser visuell erfolgen, wenn beispielsweise das Element oder seine Verbindung vollständig vom Organismus aufgenommen wurde und damit die Beschichtung verschwunden ist. Eine visuelle Auswertung kann auch noch halbquantitativ erfolgen. Die Bestimmung, insbesondere die quantitative Bestimmung, kann aber auch spektrometrisch erfolgen, beispielsweise über eine Bestimmung der verbleibenden Dicke der Beschichtung. Zur Messung kann die gesamte beschichtete Fläche des Trägers mit einem geeigneten Spektrometer gescannt werden. Die Messung kann in Transmission oder in Reflexion erfolgen. Gegebenenfalls können Durchführung und/oder Auswertung der Messung von einem Computer durchgeführt oder unterstützt werden.

Bei den als Beschichtung verwendeten Elementen kann es sich vorzugsweise um sogenannte Makro- oder um sogenannte Spurenelemente handeln. Makroelemente sind Elemente, die den größten Anteil an der mineralischen Substanz von Lebensmitteln besitzen, und an denen der Organismus einen hohen Bedarf besitzt. Makroelemente sind beispielsweise Calcium, Phosphor, Kalium, Chlor, Natrium und Magnesium. Als Spurenelemente werden bekanntlich eine Reihe von Elementen bezeichnet, die der menschliche, tierische oder pflanzliche Organismus nur in "Spuren" enthält. Diese Elemente erfüllen aber oftmals lebenswichtige Funktionen innerhalb des Organismus. Bekanntlich nehmen viele dieser Makro- oder Spurenelemente aktiv an wichtigen biochemischen Vorgängen teil. So ist beispielsweise Kupfer Bestandteil von mindestens 30 kupferhaltigen Eiweißen und Enzymen, Molybdän ist als Co-Faktor in den Enzymen Aldehyd-Dehydrogenase, Sulfit-Oxydase und Nitrat-Reduktase bekannt und Selen spielt beispielsweise in bestimmten Enzymen eine entscheidende Rolle bei bestimmten Entgiftungsprozessen im menschlichen Organismus. Besonders wichtige Elemente, aus denen nach der Erfindung Beschichtungen auf dem Trägermaterial hergestellt sein können, sind Metalle oder Elemente, die zumindest teilweise einen metallischen Charakter aufweisen. Diese Metalle oder Elemente mit zumindest teilweise metallischem Charakter gehören meist wiederum zu den Makro- oder Spurenelementen.

Elemente, die nach der Erfindung bevorzugt sind, sind: Magnesium, Aluminium, Chrom, Mangan, Eisen, Cobalt, Nickel, Kupfer, Zink, Germanium, Selen, Molybdän, Silber, Wismut, Blei oder Gold. Als weitere wichtige Elemente lassen sich beispielsweise Lithium, Titan, Thallium, Vanadium und Cadmium nennen. Auch die Elemente Calcium, Iod, Silicium, Strontium, Fluor, Zinn, Arsen und Bor kommen als wichtige Beschichtungselemente in Frage.

Wie die obigen Definitionen zeigen, gehören bevorzugt verwendbare Elemente zu den sogenannten Übergangsmetallen.

Zu diagnostischen Zwecken können entweder alle aufgeführten Elemente oder zielgerichtet nosologisch-syndromale Reihen sowie Einzelelemente angewendet werden. Eine spezielle Abstimmung auf Elementmangelerscheinungen der meisten Entzündungs-, Gefäß- und Tumorkrankheiten sowie toxischer und erblich-degenerativer Krankheiten ist möglich.

Ferner können beispielsweise spezielle Reihen für die Bewertung des Mangels an metallischen Elementen und/oder Makro- und Spurenelementen bei Diabetes, Ischämie, Immuninsuffizienz, Schmerzsyndrom, bei Störungen des Systems der Blutgerinnung (Blutkoagulation), Ulkus, Ekzem, Störungen des hormonellen Gleichgewichts und Störungen der Regenerationsfunktion bei Knochenbrüchen aufgestellt bzw. ermittelt werden.

Üblicherweise wird das als Beschichtung auf das Trägermaterial aufgebrachte Element in elementarer Form, insbesondere in reiner Form verwendet. Die Beschichtung kann jedoch auch aus einer Verbindung des Elements bestehen. Dies ist insbesondere dann von Vorteil, wenn das Element in elementarer Form instabil, beispielsweise zu reaktionsfreudig ist. Bei der Verbindung kann es sich um kovalente oder um ionische Verbindungen handeln. Es ist bevorzugt, wenn das Element in Form eines seiner Salze oder als Komplex vorliegt. So kann beispielsweise das Element Lithium in Form eines seiner anorganischen Salze und Magnesium als Magnesiumoxid in der Beschichtung vorliegen.
Besteht die Beschichtung aus der Verbindung eines Elements, so ist es bevorzugt, wenn das Element in genau dieser Verbindung auch in dem Organismus vorkommt, auf den der beschichtete Träger appliziert wird. Auf diese Weise kann davon ausgegangen werden, daß der Organismus die verwendete Verbindung verträgt, und somit besonders zuverlässige Ergebnisse erhalten werden.

Die Dicke der Beschichtung auf dem Trägermaterial ist je nach Element und zu diagnostizierender oder zu therapierender Erkrankung in weiten Grenzen variierbar. So kann die Dicke der Beschichtung zwischen 0,1 nm und 1 mm, insbesondere zwischen 0,01 µm und 100 µm, bevorzugt zwischen 0,05 µm und 30 µm betragen. In besonderen Fällen sind auch geringere Dicken als 0,1 nm anwendbar.

In Weiterbildung kann sich auf dem Träger eine Beschichtung aus nur einem Element oder aus nur einer Verbindung dieses Elements befinden. Auf diese Weise liegt für jedes Element ein eigener Träger vor. Träger mit Beschichtungen aus nur einem Element sind einfacher und billiger herstellbar als solche, bei denen mehrere Elemente nebeneinander auf einem Träger vorliegen.

Weiterhin ist es bevorzugt, wenn ein oder mehrere Elemente auf dem Trägermaterial in verschiedenen Schichtdicken aufgebracht sind. Dabei liegen insbesondere Bereiche verschiedener Schichtdicke abgegrenzt voneinander vor, so daß stufenweise auf einem Träger verschiedene Schichtdicken und damit verschiedene Mengen an Elementen vorhanden sind. Es ist bevorzugt, wenn 2 bis 5, insbesondere 3 verschiedene Schichtdicken des Elements bzw. seiner Verbindung aufgebracht sind. Anhand solcher Träger kann auf vereinfachte Weise bestimmt werden, welche Beschichtungen abgebaut und damit welche Mengen an Element vom Organismus aufgenommen wurden. So kann die prozentuale Abnahme jeder Schichtdicke beispielsweise durch eine optische Nachweismethode bestimmt werden. Auf diese Weise läßt sich qualitativ, halbquantitativ oder quantitativ bestimmen, welches Element beziehungsweise welche Mengen davon vom Organismus aufgenommen wurden. Durch die Maßnahme, daß verschiedene Schichtdicken vorgesehen sind, kann auch eine optimierte Schichtdicke, d. h. eine optimierte Dosierung der Elemente für verschiedene Anwendungen in Diagnose und/oder Therapie bestimmt werden.

Die Erfindung betrifft darüber hinaus einen Satz von beschichteten Trägern aus mindestens zwei beschichteten Trägern wie sie oben beschrieben sind. Dabei kann jeder der im Satz vorhandenen Träger eines oder mehrere Elemente oder eine oder mehrere Verbindungen dieser Elemente, gegebenenfalls in verschiedenen Schichtdicken, als Beschichtung aufweisen. Mit einem solchen Satz von beschichteten Trägern kann zur Diagnose oder Therapie von Erkrankungen im Organismus die Aufnahme eines ganzen Spektrums von Elementen durch diesen Organismus detektiert werden. Ein Satz von beschichteten Trägern, bei dem jeder Träger nur ein Element oder eine seiner Verbindungen gegebenenfalls in verschiedenen Schichtdicken aufweist, ist nach der Erfindung bevorzugt.

Weiterhin betrifft die Erfindung medizinische Diagnostika und medizinische Therapeutika, die mindestens einen der beschriebenen beschichteten Träger oder einen Satz von beschichteten Trägern, wie er oben beschrieben ist, aufweisen. Solche Diagnostika oder Therapeutika können in beliebiger Weise ausgestaltet sein, die zur Applikation an dem zu untersuchenden oder zu behandelnden Organismus geeignet ist. In einem einfachen Fall kann das Diagnostikum oder Therapeutikum aus einem Klebeband oder Pflaster bestehen, auf das die beschichteten Träger oder ein bestimmter Satz solcher Träger aufgebracht ist. Zweckmäßig werden einseitig beschichtete Träger, wie beispielsweise Plättchen, mit der nicht beschichteten Seite auf das Klebeband oder Pflaster aufgebracht. Selbstverständlich ist auch jedes andere, insbesondere einfach zu applizierende Material für diesen Zweck geeignet.

Weiter betrifft die Erfindung die Verwendung des beschriebenen beschichteten Trägers und die Verwendung des beschriebenen Satzes von beschichteten Trägern zur Diagnose und/oder Therapie von Erkrankungen eines Organismus. Von besonderer Bedeutung sind hierbei Verwendungen zur Diagnose und/oder Therapie von Erkrankungen des menschlichen Organismus.

Schließlich betrifft die Erfindung noch die Verwendung des beschriebenen beschichteten Trägers oder des beschriebenen Satzes von beschichteten Trägern zur Herstellung medizinischer Diagnostika oder Therapeutika.

Die Erfindung hat, wie bereits beschrieben, den Hintergrund, daß Erkrankungen diagnostiziert und therapiert werden sollen. Die Vorteile der Erfindung zeigen sich dementsprechend bei diesen diagnostischen und therapeutischen Methoden. Die Erfindung ist insbesondere zur Frühdiagnostik von Erkrankungen, zur präklinischen Diagnostik und zur anschließenden Korrektur von Erkrankungen, die mit einem Mangel an bestimmten Elementen im Organismus insbesondere im menschlichen Organismus zusammenhängen, geeignet.
Dabei kann versucht werden, ein nicht bekanntes Krankheitsbild zu diagnostizieren, indem eine Vielzahl beschichteter Träger oder ein entsprechender Satz von Trägern appliziert wird und anschließend die erhaltenen Ergebnisse (Mangel an bestimmten Elementen) ausgewertet werden. Aufgrund dieser Ergebnisse werden Aussagen über die vorliegende Krankheit möglich. Eine andere Möglichkeit besteht darin, eine vermutete oder durch andere Diagnoseverfahren bestimmte Krankheit durch die Erfindung zu verifizieren. Dazu wird untersucht, ob bei dem betreffenden Organismus die gleichen Mangelerscheinungen an bestimmten Elementen auftreten, wie sie bereits von anderen Organismen, die diese Erkrankung definitiv haben, auftraten. Auf diese Weise kann anhand empirisch untersuchter Krankheitsbilder der Verdacht auf eine bestimmte Erkrankung bestätigt oder verworfen werden.
Schließlich ist mit der Erfindung auch eine Therapie von Mangelerscheinungen an bestimmten Elementen möglich. Zu diesem Zweck wird dem Organismus so lange das entsprechende Element zugeführt, bis keine Aufnahme mehr erfolgt und die Bilanz im Organismus bezüglich dieses Elementes ausgeglichen ist.

Üblicherweise werden die beschichteten Träger nach der Erfindung mit Hilfe eines Klebebandes auf bestimmte Stellen des Organismus, beispielsweise bestimmte Körperstellen des Menschen appliziert. Ein Aufbringen auf jeden beliebigen Bereich der Haut und der Schleimhäute einschließlich Mundhöhle, Zunge und Bindehaut ist möglich. Die Applikatoren können auch nach den Gesichtspunkten aufgebracht werden, ob eine selektive Resorption von Elementen durch Organe erfolgt, oder ob Haut-Viszeral-Projektionswechselbeziehungen vorliegen. Bereiche zur Applikation können segmentare Bereiche, reflexogene Organzonen (nach Dr. Reinhard Voll und Dr. Stiefvater), Akupunkturmeridiane und Akupunkturpunkte sein. Dort verbleiben die beschichteten Träger für einen bestimmten Zeitraum, beispielsweise von 3 bis 5 Sekunden bei der Expressdiagnostik und bis zu 30 Tagen, beim Menschen insbesondere von 24 bis 48 Stunden. Dabei ist es vorteilhaft, wenn die Applikationszeit mindestens eine sogenannte Biorhythmusperiode durchläuft, so daß ermittelt werden kann, wie intensiv die applizierten Elemente oder Verbindungen im Verlauf des täglichen, wöchentlichen und monatlichen Biorhythmus aufgenommen werden. Auf diese Weise wird erreicht, daß Schwankungen im Stoffwechsel, die innerhalb einer solchen Biorhythmusperiode auftreten, von der Applikation abgedeckt und damit ausgeglichen werden. Nach Ablauf der Zeit wird das Klebeband entfernt und die Aufnahme der Elemente durch den Körper bzw. die Abnahme der Schichtdicke des Elementes wird, insbesondere mit Hilfe einer optischen Methode, bestimmt.

Die erfindungsgemäße Methode zur Diagnose und/oder Therapie eignet sich zur Anwendung in der ambulanten Praxis von Ärzten, in Vorsorgeeinrichtungen, in Fürsorgestellen, Sanatorien, Altenheimen, in der Sportmedizin, als präventive Therapie, bei Untersuchungskommissionen zur Bestimmung der Arbeits-, Verhandlungs- oder Verwendungsfähigkeit etc. Das Verfahren nach der Erfindung ist nicht invasiv, d. h. es tritt nicht in Kontakt mit dem direkten Blutkreislauf. Es ist auch bei Massenuntersuchungen durch Expressdiagnostik effektiv, kostengünstig und liefert sehr präzise Resultate. Eine Schädigung des Organismus erfolgt bei sachgerechter Anwendung nicht.

Zur Beschichtung des Trägermaterials mit dem betreffenden Element sind prinzipiell alle Beschichtungsverfahren geeignet, bei der die Beschichtung nach dem Aufbringen eine homogene Oberfläche aufweist, aus der der Stoff leicht, beispielsweise über die menschliche Haut, resorbiert werden kann. Verfahren, bei denen das Element der Beschichtung in das Trägermaterial in nicht resorbierbarer Form eingebracht wird, wie beispielsweise Ionenimplantation, sind nur bedingt geeignet. Geeignet sind je nach zu beschichtendem Element beispielsweise das CVD-Verfahren (Chemical Vapour Deposition), Vakuumaufdampfverfahren und elektrolytisches oder chemisches Abscheiden. Auch Pulverbeschichtungsverfahren und Beschichtung durch Sintern können anwendbar sein.

Die beschriebenen Merkmale und weitere Merkmale der Erfindung ergeben sich aus der nachfolgenden Beschreibung der Beispiele in Verbindung mit den Unteransprüchen. Hierbei können die einzelnen Merkmale jeweils für sich oder zu mehreren in Kombination miteinander verwirklicht sein.

### Beispiel 1

Eine 49jährige Patientin leidet seit fast 20 Jahren an einem Zwölffingerdarmgeschwür (Ulcus duodeni). Bei einer kürzlich eingetretenen Verschlechterung litt die Patientin nachts unter sehr starken Schmerzen der linken oberen Bauchhälfte mit Ausstrahlungen nach oben und mit dumpfen Schmerzen in der rechten Bauchregion. Durch eine Magenspiegelung wurde ein Geschwür von bis zu 0,5 cm in der unteren Wand mit einer moderaten Entzündung um den Krankheitsherd (Perifocalinflammation) diagnostiziert. Die Messung des pH-Wertes ergab eine Basal-Azidogenese des Magens im Bereich von 1,3 bis 1,5. Der Atropin-Test verlief negativ. Eine leichte Steigerung der Magen-Azidität liegt vor.
Aufgrund der Untersuchungen wurde die folgende Diagnose gestellt: Geschwür des Bulbus duodeni, ausgeprägte Bulbitis, narbige Deformation des Pylorobulbarbereiches.

Wegen einer leichten Hypersekretion des Mageninhaltes wurde eine Diagnose unter Zugrundelegung der Erfindung durchgeführt.

Auf den Projektionsbereich des Zwölffingerdarms wurde über einen Zeitraum von 24 Stunden in der Gegend des rechten Hypochondriums ein Applikator (Diagnostikum) mit fünf beschichteten Trägern gelegt. Die Träger enthielten das aufgeschichtete Element jeweils in drei Dickenabstufungen. Die Extremwerte der Dicke betrugen:

| | |
|---|---|
| - für Eisen (Fe) | 0,2 - 10 µm, |
| - für Aluminium (Al) | 0,06 - 2 µm, |
| - für Kupfer (Cu) | 0,1 - 10 µm, |
| - für Nickel (Ni) | 0,01 - 10 µm und |
| - für Chrom (Cr) | 0,01 - 1 µm. |

Als pathogenetische Grundlage für die Diagnose dienten die folgenden Kriterien, die aufgrund empirischer Bestimmungen bereits bekannt waren:
1. ein verringerter Eisengehalt im Organismus bei unveränderten Werten für Aluminium deutet auf eine verringerte Azidität des Magens;
2. ein verringerter Aluminiumgehalt im Organismus bei unveränderten Werten für Eisen deutet auf eine erhöhte Azidität des Magens;
3. ein Mangel der drei Metalle Kupfer, Nickel und Chrom im Organismus deutet auf eine aktivierte Steigerung (Exazerbation) des ulzerösen Prozesses.

Nach Abnahme des Applikators (Diagnostikums) wurde bei allen beschichteten Trägern für jedes Metall das "Maß des Verschwindens" (entspricht der Stärke der Resorption) bestimmt. Es wurden folgende Resultate erhalten, ausgedrückt in Prozent der Element(Metall-)absorption von der minimalen bis zur maximalen ursprünglichen Schichtdicke:

| | | | |
|---|---|---|---|
| Fe | 0 % | 0 % | 0 % |
| Al | 80 % | 20 % | 0 % |
| Cu | 100 % | 50 % | 5 % |
| Ni | 80 % | 40 % | 0 % |
| Cr | 90 % | 60 % | 20 % |

Unter Zugrundelegung der oben genannten Kriterien ergibt sich damit folgende Bewertung: leicht erhöhte Azidität des Mageninhalts, gemäßigte Exazerbation des ulzerösen Prozesses.

Da der Magen-Darmtrakt des Patientin in einem relativ guten Zustand war, wurde eine Korrektur des Mangels an Kupfer, Chrom, Nickel und Aluminium durch orale Einnahme von metallhaltigen Präparaten vorgenommen.

### Beispiel 2

Ein 67jähriger Patient klagt über Kopfschmerzen, Geräusche im Kopf und in den Ohren, Gedächtnisschwund, Schlafstörungen und Schwächegefühl in der rechten Hand und im rechten Bein. Vor ca. vier Jahren wurde eine akute Störung der Hirndurchblutung diagnostiziert.
Der Patient hat eine zentrale Parese des VII. rechtes Paares, eine leichte rechtsseitige spastische Hemiparese und eine diffuse Hemihypästhesie.
Es wurde folgende Diagnose gestellt: Zerebral-Arteriosklerose der II. Stufe, Resterscheinungen eines auf eine Durchblutungsstörung zurückzuführenden Insults mit leichter rechtsseitiger Hemiparese.

Wegen einer leichten Hypercholesterinämie wurde eine Diagnose gemäß der Erfindung durchgeführt.

Für eine Zeitraum von 24 Stunden wurde ein Applikator (Diagnostikum) mit vier beschichteten Trägern, von denen jeder ein Element aufwies, auf den unteren Teil des Brustbeins (Sternums) aufgelegt. Jeder der vier Träger enthielt das Element in drei Dickenabstufungen. Die Extremwerte der Dicke betrugen

| | |
|---|---|
| Cu | 0,05 - 8 µm, |
| Zn | 1 - 30 µm, |
| Co | 0,04 - 2 µm, |
| Fe | 0,1 - 8 µm. |

Empirisch waren bereits folgende Kriterien für das vermutete Krankheitsbild bekannt: der Mangel an den vier Elementen Zink, Kupfer, Cobalt und Eisen im Organismus dient als indirektes Anzeichen für eine Hypercholesterinämie.

Die Resultate lauteten (in der gleichen Weise wie in Beispiel 1 angegeben):

| | | | |
|---|---|---|---|
| Zn | 100 % | 100 % | 80 %, |
| Cu | 100 % | 90 % | 40 %, |
| Co | 100 % | 80 % | 20 %, |
| Fe | 80 % | 40 % | 0 %. |

Die Bewertung des Ergebnisses ergab folgende Diagnose: Hypercholesterinämie leichten Grades.

Aufgrund der Tatsache, daß beim Patienten eine schwere Schleimhautentzündung im Magen-Darmtrakt (Gastroenterokolitis) vorlag, wurde die Korrektur des Mangels an Zink, Kupfer, Cobalt und Eisen durch Applikation von entsprechend beschichteten Trägern auf die Bereiche von Leber und Dünndarm durchgeführt.

## Patentansprüche

1. Beschichteter Träger zur Diagnose und/oder Therapie von Erkrankungen eines Organismus, bei dem ein Trägermaterial mit mindestens einem Element des Periodensystems oder einer Verbindung dieses Elements beschichtet ist und wobei das Element in elementarer oder in gebundener Form im Organismus vorkommt.

2. Träger nach Anspruch 1, dadurch gekennzeichnet, daß es sich bei dem Trägermaterial um einen flachen Gegenstand handelt, dessen Dicke im Vergleich zu seinen übrigen Abmessungen klein ist.

3. Träger nach Anspruch 1 oder 2, dadurch gekennzeichnet, daß das Trägermaterial mindestens teilweise transparent ist, wobei insbesondere das Trägermaterial aus transparentem Kunststoff gebildet ist.

4. Träger nach einem der vorhergehenden Ansprüche, dadurch gekennzeichnet, daß es sich bei dem Element um ein sogenanntes Makroelement handelt.

5. Träger nach einem der Ansprüche 1 bis 3, dadurch gekennzeichnet, daß es sich bei dem Element um ein sogenanntes Spurenelement handelt.

6. Träger nach einem der vorhergehenden Ansprüche, dadurch gekennzeichnet, daß es sich bei dem Element um ein Metall oder um ein Element mit zumindest teilweise metallischem Charakter handelt.

7. Träger nach einem der vorhergehenden Ansprüche, dadurch gekennzeichnet, daß es sich bei dem Element um Magnesium, Aluminium, Chrom, Mangan, Eisen, Kobalt, Nickel, Lithium, Cadmium, Vanadium, Titan, Zinn, Strontium, Kupfer, Zink, Germanium, Selen, Molybdän, Silber, Wismut, Blei oder Gold handelt.

8. Träger nach einem der vorhergehenden Ansprüche, dadurch gekennzeichnet, daß es sich bei dem Element um ein Übergangsmetall handelt.

9. Träger nach einem der vorhergehenden Ansprüche, dadurch gekennzeichnet, daß das Element in Form eines seiner Salze vorliegt.

10. Träger nach einem der vorhergehenden Ansprüche, dadurch gekennzeichnet, daß das Element in Form einer Verbindung vorliegt, in der es auch im Organismus vorkommt.

11. Träger nach einem der vorhergehenden Ansprüche, dadurch gekennzeichnet, daß die Dicke der Beschichtung zwischen 0,1 nm und 1 mm, insbesondere 0,05 µm und 30 µm liegt.

12. Träger nach einem der vorhergehenden Ansprüche, dadurch gekennzeichnet, daß sich auf dem Träger eine Beschichtung aus nur einem Element oder einer Verbindung dieses Elements befindet.

13. Träger nach einem der vorhergehenden Ansprüche, dadurch gekennzeichnet, daß das Element und/oder seine Verbindung auf dem Trägermaterial in verschiedenen Schichtdicken aufgebracht ist, wobei insbesondere Bereiche verschiedener Schichtdicken abgegrenzt voneinander vorliegen.

14. Träger nach Anspruch 13, dadurch gekennzeichnet, daß das Element und/oder seine Verbindung in zwei bis fünf, insbesondere drei verschiedenen Schichtdicken aufgebracht ist.

15. Satz von beschichteten Trägern aus mindestens zwei beschichteten Trägern nach einem der vorhergehenden Ansprüche.

16. Medizinisches Diagnostikum, dadurch gekennzeichnet, daß es mindestens einen beschichteten Träger nach einem der Ansprüche 1 bis 14 oder mindestens einen Satz von beschichteten Trägern nach Anspruch 15 aufweist.

17. Medizinisches Therapeutikum, dadurch gekennzeichnet, daß es mindestens einen beschichteten Träger nach einem der Ansprüche 1 bis 14 oder mindestens einen Satz von beschichteten Trägern nach Anspruch 15 aufweist.

18. Verwendung des Trägers nach einem der Ansprüche 1 bis 14 zur Diagnose und/oder Therapie von Erkrankungen eines Organismus, insbesondere des menschlichen Organismus.

19. Verwendung des Satzes von Trägern nach Anspruch 15 zur Diagnose und/oder Therapie von Erkrankungen eines Organismus, insbesondere des menschlichen Organismus.

20. Verwendung des Trägers nach einem der Ansprüche 1 bis 14 zur Herstellung eines medizinischen Diagnostikums oder Therapeutikums.

21. Verwendung des Satzes von Trägern nach Anspruch 15 zur Herstellung eines medizinischen Diagnostikums oder Therapeutikums.
